# EUROPEAN PATENT APPLICATION

(11) **EP 2 020 639 A1**
(43) Date of publication of application: **04.02.2009**
(21) Application number: 08013857.1
(22) Date of filing: 01.08.2008
(51) Int. Cl.: G06F 19/00

(54) **Measurement results managing method, system, and apparatus**

(30) Priority: 03.08.2007 JP 2007202430
(71) Applicant: SYSMEX CORPORATION, Kobe-shi, Hyogo 651-0073 (JP)
(72) Inventor: Nakahira, Masunao, Kobe-shi Hyogo 651-0073 (JP); Kawamura, Yoshiyuki, Kobe-shi Hyogo 651-0073 (JP)
(74) Representative: HOFFMANN EITLE

(57) **Abstract**

A measurement results managing apparatus for carrying out operations, comprising: receiving a first measurement order which includes a plurality of measurement items from a high-order computer; determining whether all the measurement items included in the first measurement order are measurable at the facility at which the measurement results managing apparatus is installed; when the first measurement order includes a non-measurable item that cannot be measured at the first facility, sending a second measurement order that includes the non-measurable item to an other measurement results managing apparatus for managing measurement results received from a plurality of second analyzers, and which is installed at an other facility capable of measuring the non-measurable item; and receiving a measurement result of the non-measurable item from the other measurement results managing apparatus. A measurement results managing method and system are also disclosed.

## Description

### FIELD OF THE INVENTION

The present invention relates to a measurement results managing method, measurement results managing system, and measurement results managing apparatus. The measurement results managing apparatus manages measurement results received from a plurality of analyzers.

### BACKGROUND

Conventional measurement results managing apparatuses are known which manage measurement results received from a plurality of analyzers (for example, U.S. Laid-Open Patent Publication Nos. 2005-0102166, and 2005-0131734). This measurement results managing apparatus efficiently perfumes examination operations by managing measurement results received from a plurality of analyzers within a facility such as a hospital.

Samples that require measurement by an analyzer which is not in the on-site facility are conventionally sent with a request that the examination be performed at another off-site facility, such as an examination center. Examination request systems are known to efficiently handle requests for examination (for example, Japanese Patent Publication No. 2002-7565).

According to the measurement results managing apparatuses disclosed in U.S. Laid-Open Patent Publication Nos. 2005-0102166 and 2005-0131734, the laboratory technician must prepare an examination request form to request another facility to perform an examination when a sample must be measured by an analyzer that is not installed at the on-site facility. When the examination request system disclosed in Japanese Laid-Open Patent Publication No. 2002-7565 is used, the examination request form is efficiently prepared, but the laboratory technician must determine for which measurement items an examination request is required, and must input the data for the examination request. The laboratory technician must also input the measurement results received from the examination center into the measurement results managing apparatus.

### SUMMARY OF THE INVENTION

The scope of the present invention is defined solely by the appended claims, and is not affected to any degree by the statements within this summary.
A first aspect of the present invention is a measurement results managing method, comprising: receiving a first measurement order, which includes a plurality of measurement items, from a high-order computer by a first measurement results managing apparatus, which manages measurement results sent from a plurality of analyzers and is installed at a first facility; determining whether all the measurement items included in the first measurement order are measurable at the first facility by the first measurement results managing apparatus; when a non-measurable item that cannot be measured at the first facility is included in the first measurement order, sending a second measurement order which includes the non-measurable item to a second measurement results managing apparatus which is installed at a second facility that is capable of measuring the non-measurable item; receiving the second measurement order by the second measurement results managing apparatus at the second facility; measuring the non-measurable item included in the second measurement order at the second facility; sending a measurement result of the non-measurable item from the second measurement results managing apparatus to the first measurement results managing apparatus; and receiving the measurement result of the non-measurable item by the first measurement results managing apparatus at the first facility.
A second aspect of the present invention is a measurement results managing system, comprising: a first measurement results managing apparatus for managing measurement results received from a plurality of first analyzers, and installed at a first facility; and a second measurement results managing apparatus for managing measurement results received from a plurality of second analyzers, and installed at a second facility; wherein the first measurement results managing apparatus, including a first memory under control of a first processor, the first memory storing first instructions enabling the first processor to carry out first operations, comprising: receiving a first measurement order which includes a plurality of measurement items from a high-order computer; determining whether all the measurement items included in the first measurement order are measurable at the first facility; when a non-measurable item that cannot be measured at the first facility is included in the first measurement order, sending a second measurement order which includes the non-measurable item to the second measurement results managing apparatus; and receiving, from the second measurement results managing apparatus, a measurement result of the non-measurable item included in the second measurement order; and wherein the second measurement results managing apparatus, including a second memory under control of a second processor, the second memory storing second instructions enabling the second processor to carry out second operations, comprising: receiving the second measurement order; receiving a measurement result of the non-measurable item included in the second measurement order from at least one of the second analyzer; and sending the measurement result of the non-measurable item to the first measurement results managing apparatus.
A third aspect of the present invention is a measurement results managing apparatus for managing measurement results sent from a plurality of first analyzers, including a memory under control of a processor, the memory storing instructions enabling the processor to carry out operations, comprising: receiving a first measurement order which includes a plurality of measurement items from a high-order computer; determining whether all the measurement items included in the first measurement order are measurable at the facility at which the measurement results managing apparatus is installed; when the first measurement order includes a non-measurable item that cannot be measured at the first facility, sending a second measurement order that includes the non-measurable item to an other measurement results managing apparatus for managing measurement results received from a plurality of second analyzers, and which is installed at an other facility capable of measuring the non-measurable item; and receiving a measurement result of the non-measurable item from the other measurement results managing apparatus.
A fourth aspect of the present invention is a measurement results managing apparatus for managing measurement results sent from a plurality of analyzers, including a memory under control of a processor, the memory storing instructions enabling the processor to carry out operations, comprising: receiving a measurement order, which includes a non-measurable item that cannot be measured at an other facility, from an other measurement results managing apparatus of the other facility; receiving a measurement result of the non-measurable item included in the measurement order from an analyzer capable of measuring the non-measurable item; and sending the measurement result of the non-measurable item to the other measurement results managing apparatus.

### BRIEF DESCRIPTION OF THE DRAWINGS

FIG. 1 shows the layout of the measurement results managing apparatus of an embodiment of the present invention;
FIG. 2 shows the hardware configuration of the managing apparatus 3-A;
FIG. 3 shows the hardware configuration of the blood cell counter 5-A;
FIGs. 4 to 7 are partial flow charts showing the flow of the hospital information management system 1-B from the reception of the measurement order to the reception of the measurement results;
FIG. 8 shows an example of a measurement order stored in the hospital information management syste 1-B;
FIG. 9 shows the content of a measurable items list 150 which is stored in the managing apparatus 3-B;
FIG. 10 shows the content of the other facility information DB 170;
FIG. 11 shows an example of a measurement order sent to a hospital A;
FIG. 12 shows an example of a print screen which is printed by a printer 4-B, and a display screen which is displayed on a display 120;
FIG. 13 (a) to (c) show the content of order lists 160 and 160A; and
FIG. 14 is a flow chart showing the process following the reception of the measurement order in a modification of the managing apparatus 3-B.

### DETAILED DESCRIPTION OF THE PREFERRED EMBODIMENTS

The preferred embodiments of the present invention will be described hereinafter with reference to the drawings.

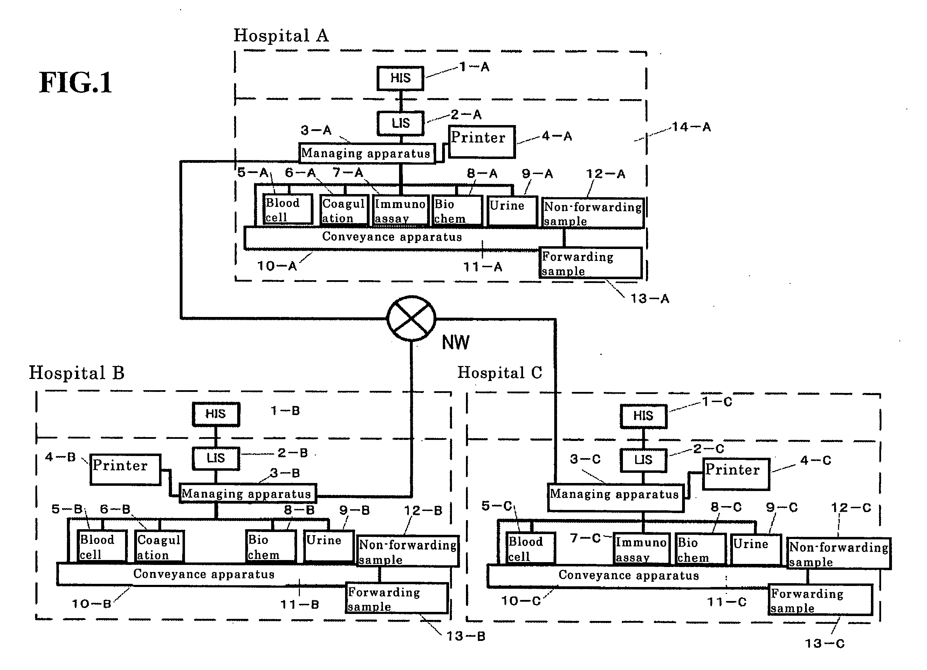

FIG. 1 shows the layout of the measurement results managing apparatus of an embodiment of the present invention. A hospital A is provided with a hospital information system (HIS) 1-A, a laboratory information system (LIS) 2-A, a measurement results managing apparatus (hereinafter referred to as "managing apparatus") 3-A, a printer 4-A, a blood cell counter 5-A, a blood coagulation measuring apparatus 6-A, an immunoanalyzer 7-A, a biochemical analyzer 8-A, a urine analyzer 9-A, and a conveyance apparatus 10-A.
Among these, the laboratory information system (LIS) 2-A, the managing apparatus 3-A, a printer 4-A, the blood cell counter 5-A, the blood coagulation measuring apparatus 6-A, the immunoanalyzer 7-A, the biochemical analyzer 8-A, the urine analyzer 9-A, and the conveyance apparatus 10-A are installed within a laboratory 14-A.

The hospital information system 1-A is a computer which manages all the operations of the hospital A, for example, storing the electronic clinical charts and the like, and receiving the measurement orders input by physicians. The measurement order includes information such as patient name, sample number, measurement items to be performed on the sample and the like, and is described later. The hospital information system 1-A is connected to the laboratory information system 2-A so that physician-input measurement orders can be sent to the laboratory information system 2-A.
The laboratory information system 2-A is a computer which manages the entire laboratory 14-A by performing earnings management, sample billing management and the like. The laboratory information system 2-A receives the measurement orders sent from the hospital information system 1-A. The laboratory information system 2-A is connected to the managing apparatus 3-A, and sends the measurement order to the managing apparatus 3-A.

The managing apparatus 3-A is a computer which manages the sample measurement results, and receives and stores the measurement orders sent from the laboratory information system 2-A. The managing apparatus 3-A is connected to a plurality of analyzers 5-A through 9-A, receives measurement results sent from the plurality of analyzers 5-A through 9-A, subjects the measurement results to validation processing and the like, and subsequently sends the measurement results to the laboratory information system 2-A. The measurement results sent to the laboratory information system 2-A are also sent to the hospital information system 1-A and reported to the physician.

The printer 4-A is connected to the managing apparatus 3-A. The printer 4-A prints address information, such as the name and address, of the other hospital on an address label to be adhered to the container which holds the sample container accommodating the sample to be shipped to the other hospital based on the information received from the managing apparatus 3-A.

The blood cell counter 5-A is a hematology analyzer which counts the number of blood cells and the amount of hemoglobin and the like in the blood; for example, a model XE-2100 (a product of Sysmex Corporation) may be used as the blood cell counter 5-A.

The blood coagulation analyzer 6-A is an analyzer which measures the blood coagulation capacity; for example, a model CS-2000i (a product of Sysmex Corporation) may be used as the blood coagulation analyzer 6-A.

The immunoanalyzer 7-A is an apparatus which measures predetermined antigens and antibodies; for example, a model PAMIA-40i (a product of Sysmex Corporation) may be used as the immunoanalyzer 7-A.

The biochemical analyzer 8-A is an apparatus which measures various substances (AST, ALT, HDL, LDL, neutral fat and the like) present in blood serum.

The urine analyzer 9-A analyzes the composition of urine; for example, a model UF-1000i (a product of Sysmex Corporation) may be used as the urine analyzer 9-A.

The conveyance apparatus 10-A transports sample containers which accommodate samples (hereinafter referred to as "samples") to the plurality of analyzers 5-A through 9-A. The conveyance apparatus 10-A is provided with a conveyor unit 11-A, a non-forwarding sample storage unit 12-A, and a forwarding sample storage unit 13-A. The conveyor unit 11-A transports samples to the plurality of analyzers 5-A through 9-A. The conveyor unit 11-A is connected to the managing apparatus 3-A, and supplies samples to the non-forwarding sample storage unit 12-A or the forwarding sample storage unit 13-A based on information from the managing apparatus 3-A. The non-forwarding sample storage unit 12-A is a device which stores the samples that have been measured for all measurement items by the analyzers 5-A through 9-A and for which all measurement results have been obtained; the forwarding sample storage unit 13-A is a device which stores samples for which all or part of the measurement results for measurement items of required examinations have not been obtained and which require examination at another facility.

A hospital B is provided with a hospital information system 1-B, a laboratory information system 2-B, a managing apparatus 3-B, a printer 4-B, a blood cell counter 5-B, a blood coagulation measuring apparatus 6-B, a biochemical analyzer 8-B, a urine analyzer 9-B, and a conveyance apparatus 10-B. An immunoanalyzer is not installed in the hospital B. Since the structures of these various apparatuses are identical to those described in hospital A, further description is therefore omitted.

A hospital C is provided with a hospital information system 1-C, a laboratory information system 2-C, a managing apparatus 3-C, a printer 4-C, a blood cell counter 5-C, an immunoanalyzer 7-C, a biochemical analyzer 8-C, a urine analyzer 9-C, and a conveyance apparatus 10-C. A blood coagulation measuring apparatus is not installed in the hospital C. Since the structures of these various apparatuses are identical to those described in hospital A, further description is therefore omitted.

The managing apparatuses 3-A, 3-B, and 3-C are connected through a network NW such as the Internet.

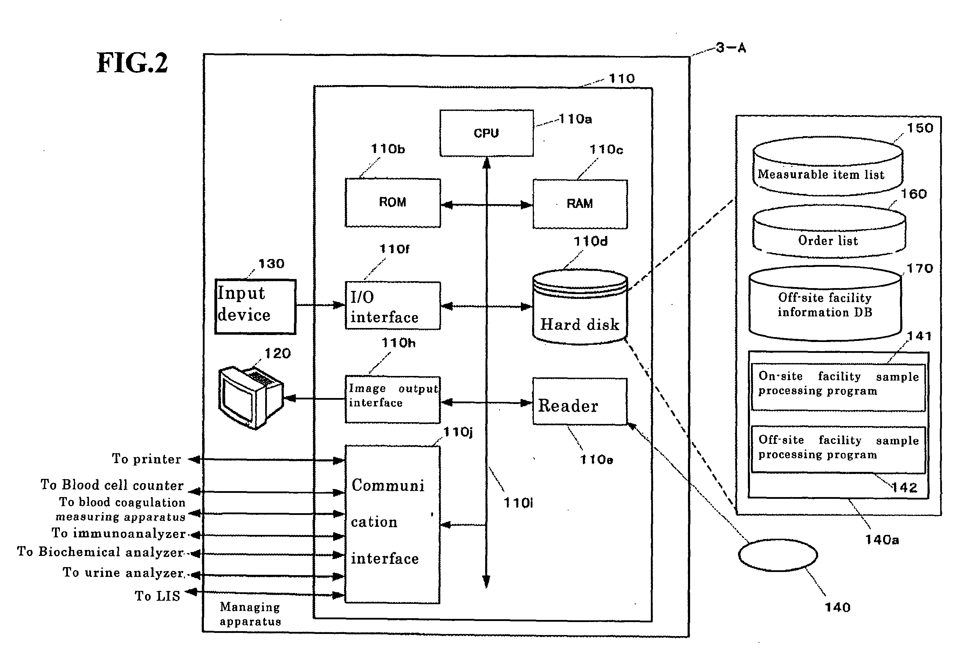

The hardware structure or the managing apparatus 3-A is described below using FIG. 2. The managing apparatus 3-A is a computer which is mainly configured by a body 110, a display 120, and an input device 130.

The body 110 is mainly configured by a CPU 110a, ROM 110b, RAM 110c, hard disk 110d, reading device 110e, input/output interface 110f, image output interface 110h, and communication interface 110j; the a CPU 110a, ROM 110b, RAM 110c, hard disk 110d, reading device 110e, input/output interface 110f, image output interface 110h, and communication interface 110j are connected by a bus 110i so as to be capable of data communication.

The CPU 110a is capable of executing computer programs stored in the ROM 110b, and computer programs loaded in the RAM 110c. The computer functions as the managing apparatus 3-A by realizing the various function blocks (described later) when the CPU 110a executes an application program 140a. The application program 140a includes an on-site sample processing program 141 for this facility and an off-site sample processing program 142 for other facilities. Details of the programs will be discussed later.

The ROM 110b is configured by a mask ROM, PROM, EPROM, EEPROM or the like, and stores computer programs executed by the CPU 110a and data and the like used in conjunction therewith.

The RAM 110c is configured by SRAM, DRAM or the like. The RAM 110c is used when reading the computer programs recorded in the ROM 110b and on the hard drive 110d.The RAM 110c is also used as a work area of the CPU 110a when the computer programs are being executed.

The hard drive 110d contains various installed computer programs to be executed by the CPU 110a such as an operating system and application program and the like, as well as data used in the execution of these computer programs.

The reading device 110e is configured by a floppy disk drive, CD-ROM drive, DVD-ROM drive or the like, and is capable of reading the computer programs and data stored on a portable recording medium 140. The portable recording medium 140 stores the application program 140a which realizes the functions of the system of the present invention on a computer, and the application 140a of the present invention is read from the portable recording medium 140 by the computer which installs the application program 140a on the hard disk 110d. FIG. 2 shows the application program 140a installed on the hard disk 110d.

The application program 140a is not only provided via the portable recording medium 304 inasmuch as the application program 140a may also be provided from an external device which is connected to the computer over an electric communication line so as to be capable of communication via this electric communication line (whether wire line or wireless). For example, when the application program 140a is stored on the hard disk of a server computer on the Internet, the managing apparatus 3-A accesses the server computer and downloads the application program 140a, which is then installed on the hard disk 110d.

An operating system which provides a graphical user interface, such as Windows (registered trademark) or the like, a product of Microsoft Corporation, USA, is installed on the hard disk 110d. The application program 140a of the present embodiment operates on this operating system in the following description.

The hard disk 110d also stores a measurable items list 150, order list 160, and off-site facility information database 170.

The input/output interface 110f is configured, for example, by a serial interface such as a USB, IEEE1394, RS232C or the like, a parallel interface such as SCSI, IDE, IEEE1284 or the like, or an analog interface such as a D/A converter, A/D converter or the like.The input device 130, which includes a keyboard and mouse, is connected to the input/output interface 110f, so that a user can input data in the managing apparatus 3-A using the input device 130.

The communication interface 110j is, for example, an Ethernet (registered trademark) interface, and via this communication interface 110j the managing apparatus 3-A sends and receives data among the various analyzers, and the managing apparatuses 3-B and 3-C which are connected the managing apparatus 3-A over the network NW using a predetermined communication protocol.

The image output interface 110h is connected to the display 120 which is configured by an LCD, CRT or the like, so that image signals corresponding to the image data received from the CPU 110a can be output to the display 120. The display 120 displays images (screens) in accordance with the input image signals.
Since the managing apparatuses 3-B and 3-C have the same structure as the managing apparatus 3-A, further description is therefore omitted.

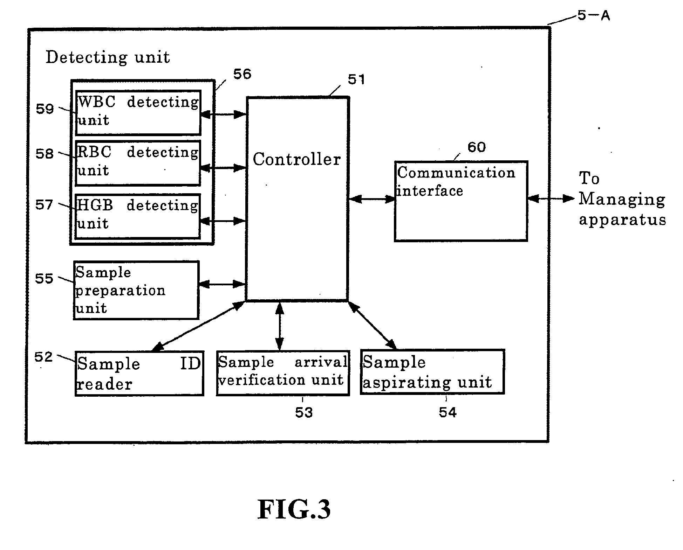

The hardware structure or the blood cell counter 5-A is described below using FIG. 3. The blood cell counter 5-A is provided with a controller 51, a sample ID reader 52, sample arrival verification unit 53, sample aspirating unit 54, sample preparing unit 55, detecting unit 56, and a communication interface 60. The controller 51 is configured by a CPU, ROM, RAM and the like, and is connected to the a sample ID reader 52, sample arrival verification unit 53, sample aspirating unit 54, sample preparing unit 55, detecting unit 56, and a communication interface 60. The controller 51 also stores a sample measurement process program which will be described later, and controls the operation of each unit, calculates the measurement results based on the detection data obtained by the detecting unit, and performs other processes such as sending the measurement results to the managing apparatus 3-A.

The sample ID reader 52 is provided with a barcode reader to read the sample ID from a barcode adhered to the sample container transported to the front of the blood cell counter 5-A by the conveyance apparatus 10-A. The barcode indicates the sample number included in the measurement order received from the hospital information system 1-A, and is adhered to the sample container before the sample container is transported by the conveyance apparatus 10-A. The sample arrival verification unit 53 is provided with a sensor (for example, a reflective-type optical sensor) to detect the presence of a sample container, at the reading position at which the barcode reader reads the barcode, or the aspirating position at which the sample is aspirated by the sample aspirating unit 54.
The sample aspirating unit 54 is provided with a pipette, pump and the like, to aspirate a sample from a sample container and supply the aspirated sample to the sample preparing unit 55.
The sample preparing unit 55 is provided with a mixing container to mix the sample and reagent, and a pump to supply the sample and the reagent to the mixing container; the sample preparing unit 55 performs processes such as diluting the sample, hemolysis and the like to prepare a detection sample.

The detecting unit 56 is provided with a white blood cell detecting unit 59, red blood cell detecting unit 58, and HGB detecting unit 57. The white blood cell detecting unit 59 is provided with a flow cytometer, and detects the white blood cells in the detection sample and sends the white cell detection data to the controller 51. The red blood cell detecting unit 58 is an electrical resistance type detector, and detects the red blood cells and the platelets in the detection sample and sends the red cell detection data to the controller 51. The HGB detecting unit 57 is provided with a light source, photoreceptor, and cell to detect hemoglobin, and sends the hemoglobin detection data to the controller 51.
The controller 51 receives the detection data (white blood cell detection data, red blood cell detection data, and hemoglobin detection data) transmitted from the detecting unit 56, and calculates the measurement results, such as the white blood cell count, red blood cell count, platelet count, and amount of hemoglobin. The calculated measurement results are then sent through the communication interface 60 to the managing apparatus 3-A.
The communication interface 60 is capable of sending and receiving data to/from the managing apparatus 3-A using a predetermined communication protocol.
The blood coagulation measuring apparatus 6-A, immunoanalyzer 7-A, biochemical analyzer 8-A, and urine analyzer 9-A are also provide with a controller, sample ID reader, sample arrival verification unit, sample aspirating unit, sample preparing unit, detecting unit, and communication interface similar to the blood cell counter 5-A.

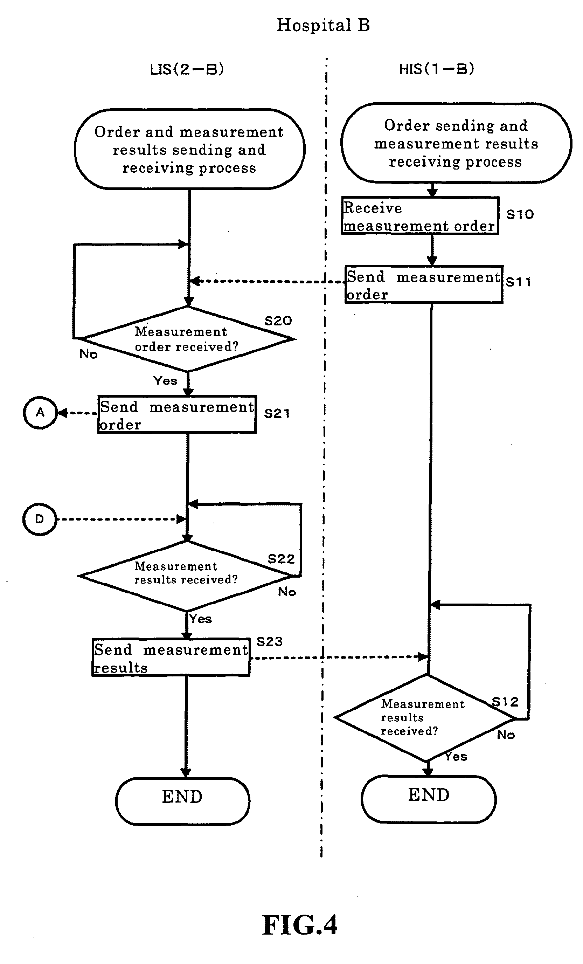

The flow from the receiving of a measurement order by the hospital information system 1-B installed at hospital B to the reception of the measurement data is described below using the flow charts of FIGS. 4 through 7.

When a physician at hospital B operates the hospital information system 1-B and inputs a measurement order, the order transmission and measurement result reception process is started. The hospital information system 1-B first executes a process to receive and store the measurement order (step S10).

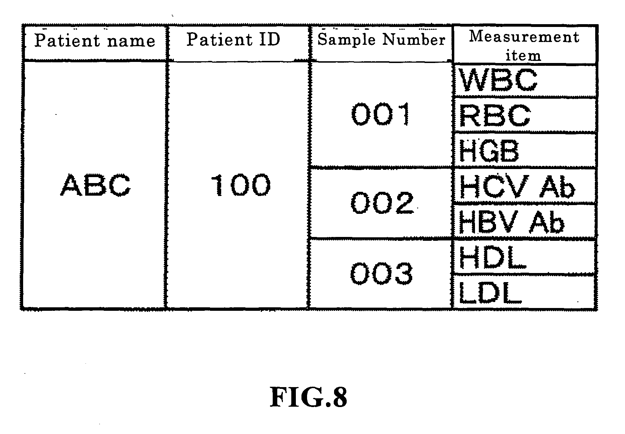

FIG. 8 shows an example of a measurement order stored in the hospital information management system 1-B. As shown in the example of FIG. 8, the measurement order includes "ABC" as the patient name; "100" as the patient ID identifying the patient "ABC," "001", "002," and "003" as sample numbers identifying the samples collected from the patient "ABC"(in this case three samples numbered "001," "002," and "003" were collected from the patient "ABC"); "WBC," "RBC," and "HGB" as sample measurement items of the sample number "001;" "HCV Ab" and "HBV Ab" as sample measurement items of the sample number "002;" and "HDL" and "LDL" as sample measurement items of the sample number "003." The "WBC," "RBC," and "HGB" sample measurement items of the sample number "001" are measurable by a blood cell counter; the "HCV Ab" and "HBV Ab" sample measurement items of the sample number "002" are measurable by a immunoanalyzer; and the "HDL" and "LDL" sample measurement items of the sample number "003" are measurable by a biochemical analyzer. "HCV Ab" represents the HCV antibody, and "HBV Ab" represents the HBV antibody.

The hospital information system 1-B then executes a process to send the measurement order to the laboratory information system 2-B b (step S11).

The laboratory information system 2-B executes a process to receive the order and measurement results, and in step S20 awaits the reception of the measurement order from the hospital information system 1-B. When the measurement order is received (step S20: YES), the laboratory information system 2-B executes a process to send the received measurement order to the managing apparatus 3-B (step S21).

The managing apparatus 3-B executes one-site sample processing for this facility. The on-site sample process is executed by the on-site sample processing program 141.

In step S30, the managing apparatus 3-B first awaits the reception of the measurement order from the laboratory information system 2-B.

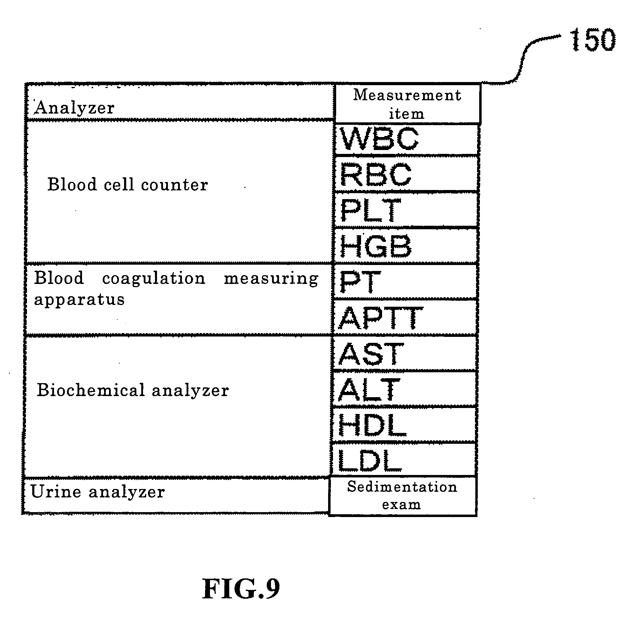

The managing apparatus 3-B then determines whether or not all measurement items included in the measurement order received in step S30 can be measured at hospital B (step S31). FIG. 9 shows the content of a measurable items list 150 (refer to FIG. 2) which is stored in the managing apparatus 3-B. As shown in the drawing, the measurable items list 150 includes a "blood cell counter," "blood coagulation measuring apparatus," "biochemical analyzer," and "urine analyzer" as analyzers; "WBC," "RBC," "PLT," "HGB" as measurement items of the blood cell counter; "PT" and "APTT" as measurement items of the blood coagulation measuring apparatus; "AST," "ALT," "HDL," and "LDL" as measurement items of the biochemical analyzer; and "sedimentation inspection (RBC, WBC, CAST and the like)" as measurement items of the urine analyzer. The measurable items list 150 is created based on the fact that "WBC," "RBC," "PLT," "HGB," "PT," "APTT," "AST," "ALT," "HDL,""LDL," and "sedimentation inspection" are measurable because the blood cell counter, blood coagulation measuring apparatus, biochemical analyzer, and urine analyzer are installed at hospital B. Although many more items are measurable by each analyzer, only the items listed above are offered in the following description to simplify the description.

In this example, the managing apparatus 3-B determines in step S31 whether or not all measurable items are included in the measurement order shown in FIG. 8, that is, whether or not all the items "WBC," "RBC," "HGB," "HCV Ab," "HBV Ab," "HDL," and "LDL" are included in the measurable items list 150. Since "HCV Ab" and "HBV Ab" are not included in the measurable items list 150 in this example, the managing apparatus 3-B determines that all measurement items included in the measurement order received in step S30 cannot be measured at hospital B.

The process advances to step S32 when it has been determined that all measurement items included in the measurement order received in step S30 can be measured at hospital B (step S31: YES).

In the present example, however, the process continues to step S33 when it has been determined that all measurement items included in the measurement order received in step S30 cannot be measured at hospital B (step S31: NO).

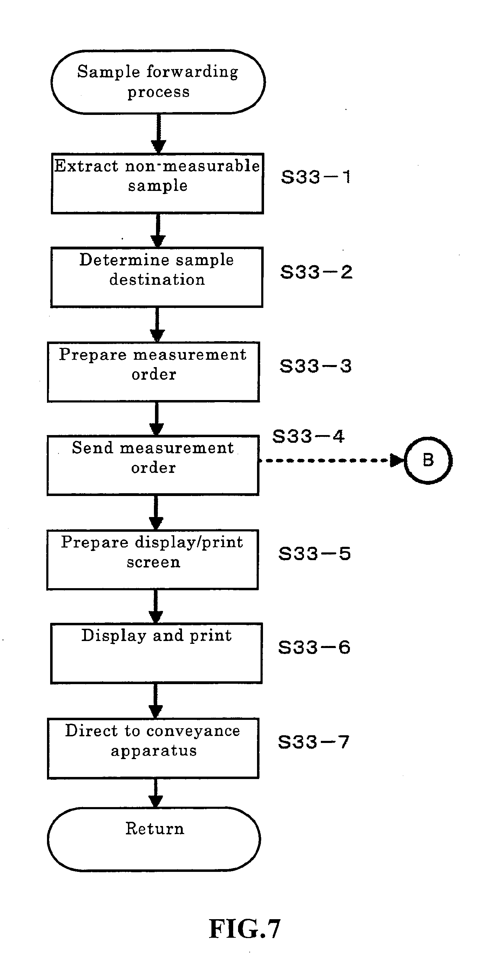

The content of the sample forwarding process of step S323 is described below using FIG. 7. The managing apparatus 3-B executes a process to extract samples which cannot be measured in step S33-1. The sample number of a sample which is designated with a measurement item that is not included in the measurable items list 150, for example the sample number "002" in the example of FIG. 8, is extracted in this process.

The managing apparatus 3-B then determines the sample forwarding destination in step S33-2. That is, the managing apparatus 3-B determines a facility which is capable of measuring the sample extracted in step S33-2.

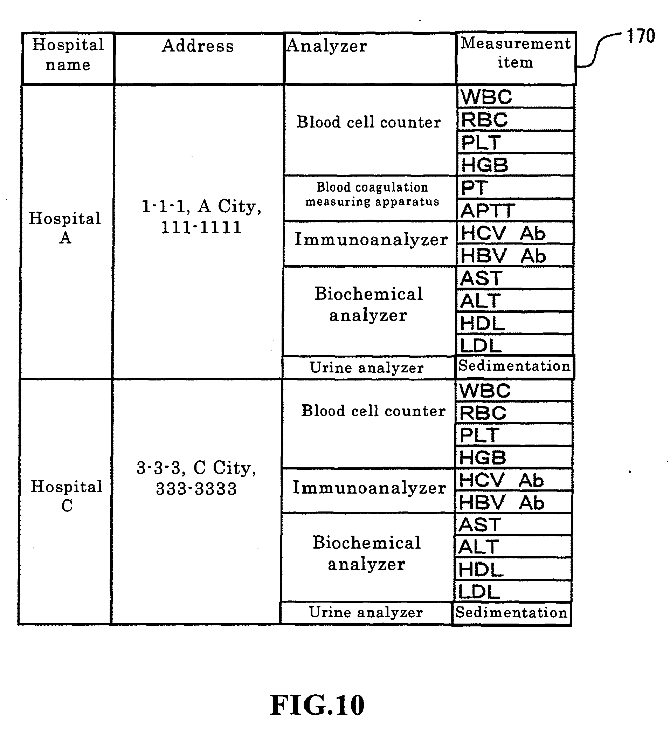

FIG. 10 shows the content of the other facility information DB 170. As shown in the drawing, the other facility information DB 170 associates and stores the hospital names, address information of the hospitals, the analyzers installed at the hospitals, and the measurement items which can be measured by these analyzers. A facility which can measure the sample is determined by determining whether or not the measurement items of the sample number extracted in step S33-1 are included in the other facility information DB 170. Since the facility to which to direct the request for the examination can be automatically determined by the managing apparatus 3-B of this configuration, the lab technician does not need to determine which facility to which to direct the request for examination, thus making the operation of requesting an examination from another facility more efficient.

In the example of a measurement order shown in FIG. 8, for example, the sample number "002" (that is, measurement items "HVC Ab" and "HBV Ab") can be measured at hospital A and hospital C. In this case, the sample forwarding destination is selected according to a predetermined rule. This rule is not specifically limited inasmuch as, for example, the hospital nearest hospital B may be selected to reduce the time of delivering the sample to the hospital.

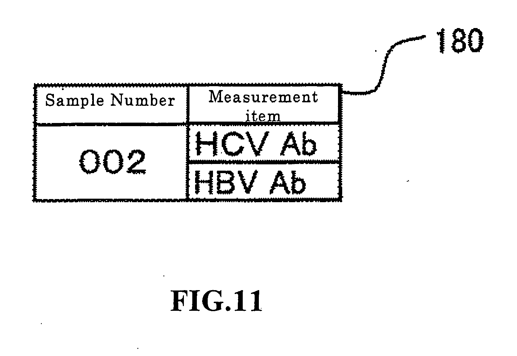

The managing apparatus 3-B then, in step S33-3, prepares the measurement order to send to the other facility (either hospital A or hospital C). FIG. 11 shows an example of a measurement order to be sent to a hospital A. That is, the sample number "002" extracted in step S33-1, and the measurement items "HCV Ab" and HBV Ab" are prepared as a measurement order 180 in step S33-3.

The managing apparatus 3-B then, in step S33-4, sends the measurement order 180 over the network NW to the managing apparatus 3-A which is installed at hospital A.

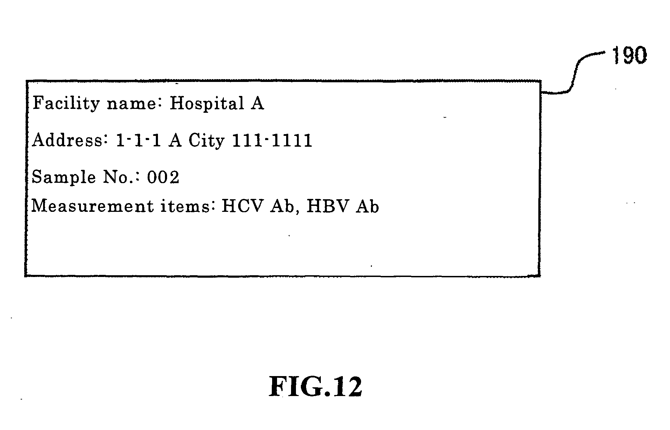

The managing apparatus 3-B also prepares a display and print screen in step S33-5. FIG. 12 shows an example of a print screen and a display screen which are prepared in step S33-5. As shown in the drawing, the display screen and the print screen 190 includes the name of the hospital at the forwarding destination of the sample, the address of the hospital, the sample number of the sample being sent, and the sample measurement items.

In step S33-6, the managing apparatus 3-B displays the display screen 190 on the display 120, and prints the print screen 190 via the printer 4-B.
In step S33-7, the managing apparatus 3-B also instructs the conveyance apparatus 10-B to transport the sample having the sample number "002," which must be sent to another off-site facility such as hospital A, to forwarding sample storage unit 13-B, and transport the samples having sample numbers "001" and "003,"which do not need to be forwarded to another off-site facility, to the non-forwarding sample storage unit 12-B. When this instruction is received, the conveyance apparatus 10-B transports the sample having the sample number "002" to the forwarding sample storage unit 13-B, and transports the samples having the sample numbers "001" and "003" to the non-forwarding sample storage unit 12-B.

After these processes, the staff at hospital B picks up the sample container which has the sample number "002" received from the forwarding sample storage unit 13-B, places this sample in a container such as a refrigerated container, adheres a form (label) bearing the printed print screen 190 in step S33-6, and mails the container to hospital A. It is thus possible in this system to perform the sample examination efficiently because the label indicating the sample forwarding destination is printed automatically, and the sample which must be forwarded to another facility is sorted automatically.

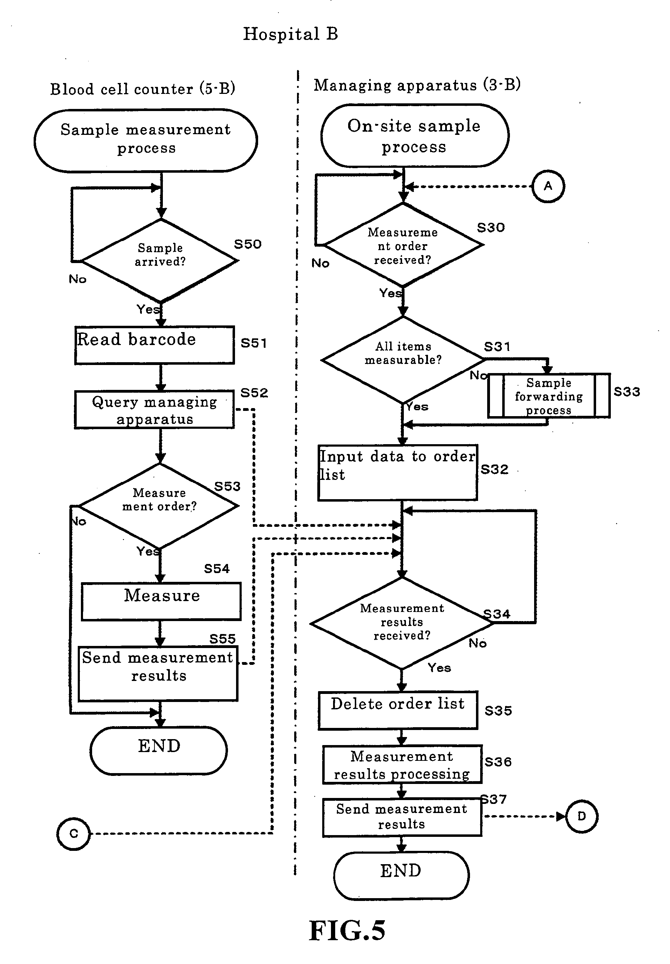

Returning now to FIG. 5, the process of step S32 is described below.

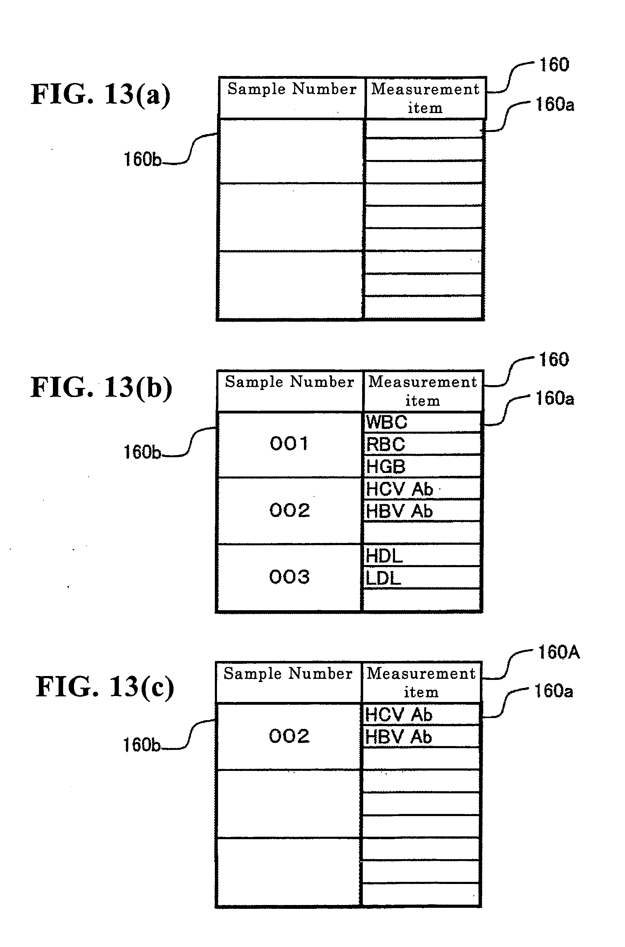

FIG. 13 shows the content of order list 160 (refer to FIG. 2). As shown in FIG. 13(a), the order list 160 includes a measurement item storage region 160a, and a sample number storage region 160b. In step S32, the managing apparatus 3-B inputs, into the order list 160, the sample numbers (in the current example, "001," "002," "003") and the measurement items (in the current example, "WBC," "RBC," "HGB," "HCV Ab," "HBV Ab," "HDL," and "LDL") from the measurement order received in step S30 (refer to FIG. 8). Thus, measurement items "WBC," "RBC," and "HGB" are associated with sample number "001;" measurement items "HCV Ab" and HBV Ab" are associated with sample number "002;" and measurement items "HDL" and "LDL" are associated with sample number "003" as shown in FIG. 13(b).

When it has been determined in step S31 that not all measurement items are measurable, the inputting of such a sample (in the current example, the sample having sample number "002"), which is included in the measurement order prepared in step S33-3, may also be omitted from the order list 160.

The analyzers 5-B through 9-B execute the sample measurement processes in parallel with the on-site sample processing performed by the managing apparatus 3-B. This process is executed by a sample measurement process program stored in the controller 51. FIG. 5 shows the content of the sample measurement process performed by the blood cell counter 5-B, as an example.

The controller 51 of the blood cell counter 5-B executes a process to confirm the sample arrival in step S50. Specifically, a process is executed to await the detection, via a sensor included in the sample arrival verification unit 53, that the sample has been transported to a barcode reading position of the sample ID reader 52 via the conveyance apparatus 10-B.

When the sample arrival is verified (step S50: YES), the controller 51 executes a barcode reading process in step S51. Specifically, a process is executed to read the sample number from a barcode adhered to the sample container by a barcode reader included in the sample ID reader 52.

In step S52, the controller 51 then executes a process to query the managing apparatus 3-B as to whether or not the sample number read in step S51 is included in the order list 160 which was prepared in step S32.

When it is determined that the sample number is included in the order list 160 (step S53: YES) as a result of the query of step S52, the controller 51 executes the measurement process in step S54. In this process the sample is aspirated from the sample container by the sample aspirating unit 54, a detection sample is prepared by the sample preparing unit 55, the white blood cells (WBC), red blood cells (RBC), and hemoglobin (HGB) are detected by the sample detecting unit 56, the detection data are sent to the controller 51, and the WBC, RBC, and HGB measurement results are calculated by the controller 51.

In step S55, the controller 51 then sends the WBC, RBC, and HGB measurement results which were calculated in step S54 to the managing apparatus 3-B, which completes the sample measurement process for this sample.

However, when it is determined that the sample number is not included in the order list 160 (step S53: NO) as a result of the query of step S52, the sample measurement process for this sample ends without executing the measurement process.

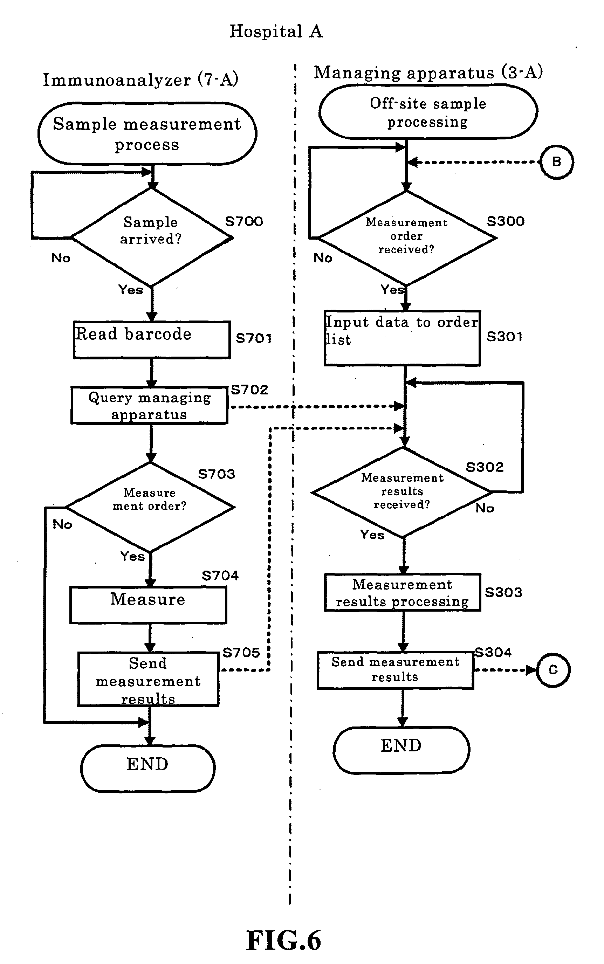

The off-site facility sample process performed by the managing apparatus 3-A, which has received the transmitted sample measurement order from the managing apparatus 3-B, is described below using FIG. 6. This process is executed by the off-site sample processing program 142 which is stored in the managing apparatus 3-A.

The managing apparatus 3-A executes a process to await the reception of a measurement order 180 from the other facility (hospital B in the above example) in step S300.

When the measurement order 180 is received from another facility (step S300: YES), the managing apparatus 3-A executes a data input process to input data in an order list 160 (hereinafter this order list is referred to as order list 160A to distinguish it from the order list 160 of the managing apparatus 3-B) of the managing apparatus 3-A. In this process, according to the above example, the sample number "002" and measurement items "HCV Ab" and HBV Ab," which are included in the measurement order 180 (refer to FIG. 11), are input in the order list 160A. FIG. 13(c) shows the content of the order list 160A into which this information has been input.

The analyzers 5-B through 9-B execute the sample measurement processes in parallel with the off-site sample processing performed by the managing apparatus 3-A. FIG. 6 shows the content of the sample measurement process performed by the immunoanalyzer 7-A.

The controller 51 of the immunoanalyzer 7-A executes a process to confirm the sample arrival in step S700. Specifically, a process is executed to await the detection, via a sensor included in the sample arrival verification unit 53, that the sample has been transported to the barcode reading position of the sample ID reader 52 via the conveyance apparatus 10-A. In the above example, this sample is the sample which has the sample number "002"that was mailed from hospital B.

When the sample arrival is verified (step S700: YES), the controller 51 executes a barcode reading process in step S701. Specifically, a process is executed to read the sample number from a barcode adhered to the sample container by a barcode reader included in the sample ID reader 52.

In step S702, the controller 51 then executes a process to query the managing apparatus 3-A as to whether or not the sample number read in step S701 is included in the order list 160A which was prepared in step S301.

When it is determined that the sample number is included in the order list 160A (step S703: YES) as a result of the query of step S702, the controller 51 executes the measurement process in step S704. In this process the sample is aspirated from the sample container by the sample aspirating unit 54, a detection sample is prepared by the sample preparing unit 55, the HCV antibody (HCV Ab) and the HBV antibody (HBV Ab) are detected by the sample detecting unit 56, the detection results are sent to the controller 51, and the HCV Ab and HBV Ab measurement results are calculated by the controller 51.

In step S705, the controller 51 then sends the HVC Ab and HBV Ab measurement results calculated in step S704 to the managing apparatus 3-A, and the sample measurement process for this sample is completed.

However, when it is determined that the sample number is not included in the order list 160A (step S703: NO) as a result of the query of step S702, the sample measurement process for this sample ends without executing the measurement process.

In step S302 the managing apparatus 3-A also executes a process to await the reception of the measurement result of the measurement items included in the order list 160A from the analyzer. Since "HCV Ab" and HBV Ab" are included in the order list 160A in the above example, the reception of those measurement result are awaited.

In step S303 the managing apparatus 3-A then executes measurement result processing such as checking whether or not re-examination is required, measurement result validation, and data format conversion and the like in order to send the measurement results to the managing apparatus 3-B. In step S304 the managing apparatus 3-A then sends the measurement data (according to the above example, these measurement results include "HCV Ab" and "HBV Ab" for sample number "002 ) to the managing apparatus 3-B.

After the process of step S303, the managing apparatus 3-A may, for example, also prepare a bill as electronic data to request an examination fee corresponding to the analyzed measurement items from hospital B, and send this bill over the network NW to the managing apparatus 3-B. Thus, the billing operation for the examination material can be performed efficiently.

In step S34 the managing apparatus 3-B executes a process to await the reception of the measurement results of all measurement items included in the order list 160 from the analyzers 5-B through 9-B and the managing apparatus 3-A.

When the measurement results of all the measurement items (in the above example, "WBC," "RBC," "HGB," "HVC Ab," "HBV Ab," "HDL, and "LDL") included in the order list 160 are received (step S34: YES), the managing apparatus 3-B executes a process to delete, from the order list 160, those measurement items for which the measurement results have been received, and sample number associated therewith in step S35.

In step S36 the managing apparatus 3-B then executes measurement result processing such as checking whether or not re-examination is required, measurement result validation, and data format conversion and the like in order to send the measurement results to the laboratory information system 2-B.

In step S37 the managing apparatus 3-B sends the measurement results (in the above example, "WBC," "RBC," and "HGB" for sample number "001;" "HCV Ab" and "HBV Ab" for sample number "002;" and "HDL" and "LDL" for sample number "003") to the laboratory information system 2-B.

In step S22 (refer to FIG. 4) the laboratory information system 2-B awaits the reception of the measurement results from the managing apparatus 3-B; and when the results are received (step S22: YES), the these measurement results are sent to the hospital information system 1-B in step S23.

In step S12 the hospital information system 1-B awaits the reception of the measurement results from the laboratory information system 2-B; and when the results are received (step S12: YES), the order transmission and measurement result reception process is completed.

According to the measurement results managing system of the present embodiment, the measurement results of the measurement items included in a measurement order are reported to the hospital information system 1-B even when all the measurement items cannot be measured at the onsite facility, and without the lab technician of hospital B performing data input processing for an examination request to another off-site facility, or inputting data to the managing apparatus for the measurement results received from another off-site facility.

Although managing apparatuses are installed in three hospitals (facilities) in the example of the present embodiment, the number of facilities is not limited insofar as there are a plurality of such facilities. Furthermore, some or all of the facilities may also be examination centers which receive requests of measuring samples from hospitals.

Although only samples which require analysis at another off-site facility (sample with sample number "002" in the example) are forwarded in the above embodiment, the present invention is not limited to this arrangement inasmuch as all the samples (in the example, samples with sample numbers "001," "002," and "003") collected from a patient for which the sample requiring analysis at another off-site facility was collected may be forwarded to an off-site facility.

Although the hospital information system, laboratory information system, and managing apparatuses are installed in hospitals in the above embodiment, the present invention is not limited to this arrangement inasmuch as the laboratory information system may also have the functions of the managing apparatus (in which case, the laboratory information system provided with the functions of the managing apparatus would be an embodiment of the present invention), and the hospital information system and the laboratory information system may be integrated and the functions of the managing apparatus may be provided in the integrated system.

Although all or some of the analyzers 5-A through 9-A are connected to the managing apparatus in the above embodiment, the number and type of analyzer connected to the managing apparatus is not limited.

Although an off-site facility is requested to perform a measurement when the on-site facility lacks the needed analyzer in the above embodiment, the present invention is not limited to this arrangement inasmuch as the present invention is also applicable to a measurement results managing system which is capable of automatically requesting measurements from an off-site facility when the analyzer is malfunctioning at the on-site facility. Such a measurement results managing system may be realized, for example, by making the measurable items list 150 editable, so that an analyzer and the measurement items performed by that analyzer can be deleted from the measurable items list 150 when the analyzer of the on-site facility is malfunctioning. Such data deletion may also be performed by a user of the measurement results managing system, or may be automatically performed based on the information from the malfunctioning analyzer.

Although three managing apparatuses are connected over a network NW in the above embodiment, the present invention is not limited to this arrangement inasmuch as the managing apparatuses and the hospital information system or the laboratory information system may also be connected over the network NW. In this case the measurement order and the measurement results may be sent from the managing apparatus to another managing apparatus through the hospital information system or the laboratory information system.

Although the printer 4-B prints the print screen 190 on a label and this label is adhered to a container which contains the sample by the staff of hospital B in the above embodiment, the present invention is not limited to this arrangement inasmuch as the printer 4-B may also print the print screen 190 directly on the container containing the sample (for example, an envelope containing the sample).

Although the managing apparatus 3-B awaits the reception of the measurement results for all measurement items included in the order list 160 in step S34 of the above embodiment, the present invention is not limited to this arrangement inasmuch as the measurement results may also be sent to the laboratory information system each time a predetermined measurement item is received. For example, when the measurement results of the measurement items analyzed at hospital B (WBC, RBC, HGB, HDL, LDL) are received from the analyzers, these measurement results may be sent to the laboratory information system 2-B, and later when the measurement results of the measurement items (HCV Ab, HBV Ab) are received from the managing apparatus 3-A, these measurement results may be sent to the laboratory information system 2-B.

Although the managing apparatus is provided with both an on-site sample processing program 141 and an off-site sample processing program 142 in the above embodiment, the present invention is not limited to this arrangement inasmuch as only one such program may also be provided. For example, the above described sample measurements can be measured when the managing apparatus 3-B of hospital B is provided with only the on-site sample processing program 141, and the managing apparatus 3-A of hospital A is provided with only the off-site sample processing program 142.

Although the managing apparatus determines the hospital from which to request an examination in step S33-2 of the above embodiment, the present invention is not limited to this arrangement inasmuch as the hospital from which to request an examination may be predetermined, so that the process of step S33-2 may be omitted by always sending the measurement order to that predetermined hospital in step S33-4.

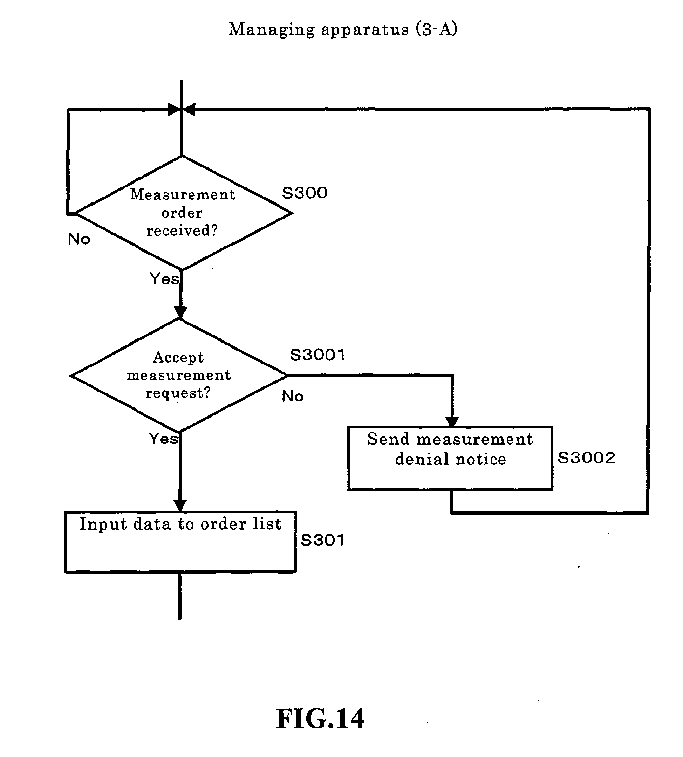

Although the managing apparatus 3-A of hospital A is configured to always execute the measurements of measurement items included in the measurement order when a measurement order is received from another facility in the above embodiment, the present invention is not limited to this arrangement inasmuch as the managing apparatus 3-A may also be allowed to select whether or not execute the measurement of the requested measurement items. As shown in FIG. 14, for example, when a measurement order is received from another facility in step S300 (step S300: YES), the managing apparatus 3-A determines whether or not to agree to the measurement request in step S3001; and when agreement is determined (step S3001: YES), executes a data input process to input data to the order list in step S301. The determination as to whether or not to agree in step S3001 may be based on input from a user of the managing apparatus 3-A, an accepting facility may be determined beforehand, and acceptance by be determined based on the number of sample processes performed at hospital A that day. However, when denial of the measurement request is determined in step S3001 (step S3001: NO), a measurement denial notice is sent to the originating managing apparatus of the measurement order in step S3002 and the process returns to step S300 without inputting data in the order list.

## Claims

1. A measurement results managing method, comprising:
receiving a first measurement order, which includes a plurality of measurement items, from a high-order computer by a first measurement results managing apparatus, which manages measurement results sent from a plurality of analyzers and is installed at a first facility;
determining whether all the measurement items included in the first measurement order are measurable at the first facility by the first measurement results managing apparatus;
when a non-measurable item that cannot be measured at the first facility is included in the first measurement order, sending a second measurement order which includes the non-measurable item to a second measurement results managing apparatus which is installed at a second facility that is capable of measuring the non-measurable item;
receiving the second measurement order by the second measurement results managing apparatus at the second facility;
measuring the non-measurable item included in the second measurement order at the second facility;
sending a measurement result of the non-measurable item from the second measurement results managing apparatus to the first measurement results managing apparatus; and
receiving the measurement result of the non-measurable item by the first measurement results managing apparatus at the first facility.

2. The measurement results managing method of claim 1, further comprising:
outputting address information of the second facility from the first measurement results managing apparatus when the non-measurable item is included in the first measurement order.

3. The measurement results managing method of claim 1, further comprising:
directing one of the analyzers at the first facility to perform measurement of measurable items included in the first order by the first measurement results managing apparatus;
receiving measurement results of the measurable items from the analyzer which received the direction to measure the measurable items by the first measurement results managing apparatus; and
sending the measurement results of the measurable items from the first measurement results managing apparatus to the high-order computer.

4. The measurement results managing method of claim 1, further comprising:
determining whether to accept measurement of the non-measurable item included in the second measurement order when the second measurement results managing apparatus has received the second measurement order from the first measurement results managing apparatus.

5. The measurement results managing method of claim 4, further comprising:
sending a measurement denial notice to the first measurement results managing apparatus from the second measurement results managing apparatus when the second measurement results managing apparatus determines to deny the measurement of the non-measurable item.

6. A measurement results managing system, comprising:
a first measurement results managing apparatus for managing measurement results received from a plurality of first analyzers, and installed at a first facility; and
a second measurement results managing apparatus for managing measurement results received from a plurality of second analyzers, and installed at a second facility; wherein
the first measurement results managing apparatus, including a first memory under control of a first processor, the first memory storing first instructions enabling the first processor to carry out first operations, comprising:
receiving a first measurement order which includes a plurality of measurement items from a high-order computer;
determining whether all the measurement items included in the first measurement order are measurable at the first facility;
when a non-measurable item that cannot be measured at the first facility is included in the first measurement order, sending a second measurement order which includes the non-measurable item to the second measurement results managing apparatus; and
receiving, from the second measurement results managing apparatus, a measurement result of the non-measurable item included in the second measurement order; and wherein
the second measurement results managing apparatus, including a second memory under control of a second processor, the second memory storing second instructions enabling the second processor to carry out second operations, comprising:
receiving the second measurement order;
receiving a measurement result of the non-measurable item included in the second measurement order from at least one of the second analyzer; and
sending the measurement result of the non-measurable item to the first measurement results managing apparatus.

7. A measurement results managing apparatus for managing measurement results sent from a plurality of first analyzers, including a memory under control of a processor, the memory storing instructions enabling the processor to carry out operations, comprising:
receiving a first measurement order which includes a plurality of measurement items from a high-order computer;
determining whether all the measurement items included in the first measurement order are measurable at the facility at which the measurement results managing apparatus is installed;
when the first measurement order includes a non-measurable item that cannot be measured at the first facility, sending a second measurement order that includes the non-measurable item to an other measurement results managing apparatus for managing measurement results received from a plurality of second analyzers, and which is installed at an other facility capable of measuring the non-measurable item; and
receiving a measurement result of the non-measurable item from the other measurement results managing apparatus.

8. The measurement results managing apparatus of claim 7, further comprising an output device, wherein the memory stores address information of the other facility, and wherein the operations further comprise:
extracting the address information of the other facility from the memory when the non-measurable measurement item is included in the first measurement order; and
outputting the extracted address information from the output device.

9. The measurement results managing apparatus of claim 8, wherein
the output device comprises a printer for printing the address information on a container for forwarding a sample, which is to be measured for the non-measurable item, to the other facility or a label to be adhered to the container.

10. The measurement results managing apparatus of any one among claims 7 through 9, wherein the operations further comprise:
awaiting reception of measurement results sent from the plurality of first analyzers, and the measurement result of the non-measurable item sent from the other measurement results managing apparatus; and
sending the measurement results sent from the plurality of first analyzers and the measurement result of the non-measurable item sent from the other measurement results managing apparatus.

11. The measurement results managing apparatus of any one among claims 7 through 10, wherein
the measurement results managing apparatus is connected to a conveyance apparatus for conveying samples to the plurality of first analyzers, and wherein the operations further comprise:
instructing sample conveyance destination to the conveyance apparatus based on existence of the non-measurable item included in the first measurement order.

12. The measurement results managing apparatus of any one among claims 7 through 11, wherein
the determination step comprises a step of determining that at least one measurement item included in the first measurement order cannot be measured at the facility at which the measurement results managing apparatus is installed when none of analyzer capable of measuring the non-measurable item is installed at the facility at which the measurement results managing apparatus is installed.

13. The measurement results managing apparatus of any one among claims 7 through 12, wherein
the determination step comprises a step of determining that at least one measurement item included in the first measurement order cannot be measured at the facility at which the measurement results managing apparatus is installed when an analyzer capable of measuring the non-measurable item is malfunctioning.

14. The measurement results managing apparatus of any one among claims 7 through 13, wherein the operation further comprises:
a step of selecting the other facility capable of measuring the non-measurable item from among a plurality of facilities capable of measuring the non- measurable item.

15. A measurement results managing apparatus for managing measurement results sent from a plurality of analyzers, including a memory under control of a processor, the memory storing instructions enabling the processor to carry out operations, comprising:
receiving a measurement order, which includes a non-measurable item that cannot be measured at an other facility, from an other measurement results managing apparatus of the other facility;
receiving a measurement result of the non-measurable item included in the measurement order from an analyzer capable of measuring the non-measurable item; and
sending the measurement result of the non-measurable item to the other measurement results managing apparatus.
